(19) Europäisches Patentamt
European Patent Office
Office européen des brevets

(11) **EP 1 550 432 A2**

(12) **DEMANDE DE BREVET EUROPEEN**

(43) Date de publication:
**06.07.2005 Bulletin 2005/27**

(51) Int Cl.[7]: **A61K 7/035**, A61K 7/02

(21) Numéro de dépôt: **04292992.7**

(22) Date de dépôt: **14.12.2004**

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **19.12.2003 FR 0351125**

(71) Demandeur: **L'OREAL**
**75008 Paris (FR)**

(72) Inventeurs:
- **Hadasch, Anke**
**75013 Paris (FR)**
- **Delacour, Marie-Laure**
**75013 Paris (FR)**
- **Ray, Xavier**
**91580 Villeconin (FR)**

(74) Mandataire: **Boulard, Denis**
**L'OREAL - D.I.P.I.**
**25-29 Quai Aulagnier**
**92600 Asnières (FR)**

(54) **Composition cosmétique poudreuse comprenant un organopolysiloxane élastomère**

(57) L'invention a pour objet une composition cosmétique poudreuse comprenant de l'eau, des particules solides d'organopolysiloxane élastomère, des particules additionnelles, les particules d'organopolysiloxane élastomère et les particules additionnelles étant présentes selon un rapport pondéral organopolysiloxane élastomère/particules additionnelles supérieur à 1,2 et inférieur ou égal à 2,5.

La composition s'applique facilement sur les matières kératiniques, en particulier sur la peau et permet d'obtenir un maquillage bien réparti, homogène.

Application au maquillage et au soin des matières kératiniques, notamment de la peau.

EP 1 550 432 A2

## Description

La présente invention a pour objet une composition poudreuse, notamment cosmétique, comprenant des particules d'organopolysiloxane élastomère et des particules additionnelles. L'invention a également pour objet un procédé de maquillage ou de traitement non thérapeutique des matières kératiniques d'être humain, telles que la peau, les ongles, les cils, les sourcils, les cheveux, et en particulier de la peau, comprenant l'application de la composition sur les matières kératiniques.

La composition selon l'invention peut être une composition de maquillage ou de soin des matières kératiniques, en particulier de la peau, et de préférence une composition de maquillage.

La composition de maquillage peut être un produit de maquillage de la peau, tel qu'un produit du teint (notamment fond de teint), un fard à paupières, un eyeliner, un fard à joue, un produit anticernes, un produit de maquillage du corps, un produit de maquillage des lèvres, un produit de maquillage des ongles, un produit de maquillage des cheveux. De préférence, la composition est une composition de maquillage de la peau, en particulier un fond de teint, un fard à paupières, un fard à joues. Plus spécialement, l'invention porte sur une composition de maquillage du teint, notamment un fond de teint.

La composition de soin de la peau peut être un produit de soin de la peau (du visage,du corps, des mains), un produit matifiant de la peau, un produit de protection solaire de la peau (notamment du visage), une composition autobronzante, un produit déodorant.

Il est connu de la demande WO 02/053126 des compositions cosmétiques comprenant des particules d'organopolysiloxane réticulé élastomère en milieu aqueux et des poudres telles que des pigments et des charges, présentes selon un ratio pondérales organopolysiloxane élastomère / poudres allant de 0,4 à 1,2 ; ces compositions présentent une texture pulvérulente à pâteuse. Ces compositions pâteuses sont notamment conditionnées dans une coupelle et présentent une surface de prélèvement ayant un caractère élastique procurant ainsi un toucher original et différent de celui des poudres compactes classiques dont le toucher est dur, rigide.

Pour utiliser les compositions décrites dans la demande WO 02/053126, l'utilisatrice prélève une quantité de produit à l'aide d'un applicateur tel qu'une éponge, une houppette ou un pinceau, ou bien au doigt puis l'applique sur la matière kératinique à traiter ou à maquiller, par exemple sur la peau. Or le produit est difficile à appliquer sur la peau en raison de la teneur élevée en poudres et de la faible teneur en liquide : l'utilisatrice ressent un frein lors de l'étalement du produit car le produit sèche trop vite lors de l'application. Du fait de cette difficulté d'application, l'utilisatrice ne peut pas travailler le maquillage déposé sur la peau pour pouvoir bien l'étaler de manière régulière sur toute la surface de la peau qu'elle souhaite maquiller. Le produit est alors déposé sur la peau de manière irrégulière et le maquillage obtenu n'est pas homogène puisque des traces de produit (notamment des traces de couleur) sont visibles sur la peau.

Le but de la présente invention est donc de disposer d'une composition poudreuse comprenant des particules d'organopolysiloxane réticulé élastomère et des poudres et pouvant s'étaler facilement à l'aide d'un applicateur ou au doigt sur les matières kératiniques, en particulier sur la peau.

Les inventeurs ont découvert qu'une telle composition est obtenue en utilisant un rapport pondéral particulier entre les particules d'organopolysiloxane réticulé élastomère et les particules additionnelles. Ce rapport pondéral particulier permet d'améliorer la facilité d'application du produit sur les matières kératiniques, en particulier sur la peau. La composition s'étale aisément sur la peau et permet à l'utilisatrice de bien travailler la répartition du produit sur la peau, conduisant ainsi à l'obtention d'un maquillage homogène, bien réparti sur la peau sans laisser de traces visibles.

De façon plus précise, l'invention a pour objet une composition poudreuse comprenant de l'eau, des particules solides d'organopolysiloxane réticulé élastomère, des particules additionnelles, les particules d'organopolysiloxane réticulé élastomère et les particules additionnelles étant présentes selon un rapport pondéral organopolysiloxane réticulé élastomère/particules additionnelles supérieur à 1,2 et inférieur ou égal à 2,5. La composition est en particulier une composition cosmétique.

L'invention a également pour objet un procédé cosmétique de maquillage ou de traitement non thérapeutique des matières kératiniques, en particulier de peau, comprenant l'application sur les matières kératiniques, en particulier la peau, d'une composition telle que définie précédemment.

L'invention a aussi pour objet l'utilisation d'une composition telle que définie précédemment pour obtenir un maquillage facile à appliquer sur les matières kératiniques, en particulier sur la peau, ou un maquillage homogène déposé sur les matières kératiniques, en particulier sur la peau.

On entend par composition poudreuse une composition comprenant une phase pulvérulente et ayant une texture pouvant être sous forme de poudre libre, de composition solide (poudre compacte ou poudre pressée), ou sous forme de composition pâteuse. La composition poudreuse n'est pas une composition liquide (au sens d'une composition qui s'écoule sous son propre poids à température ambiante (25 °C) ). Avantageusement, la composition poudreuse selon l'invention est une composition solide.

On entend par composition solide une composition qui ne s'écoule pas sous son propre poids à température ambiante (25 °C) au bout d'une heure.

La composition selon l'invention contient des particules d'organopolysiloxane réticulé élastomère. L'organopolysiloxane réticulé élastomère peut être obtenu par réaction d'addition réticulation de diorganopolysiloxane contenant au moins un hydrogène lié au silicium et de diorganopolysiloxane ayant des groupements à insaturation éthylénique liés au silicium, notamment en présence de catalyseur platine ; ou par réaction de condensation réticulation déhydrogénation entre un diorganopolysiloxane à terminaisons hydroxyle et un diorganopolysiloxane contenant au moins un hydrogène lié au silicium, notamment en présence d'un organoétain ; ou par réaction de condenstaion réticulation d'un diorganopolysiloxane à terminaisons hydroxyle et d'un organopolysilane hydrolysable ; ou par réticulation thermique d'oranopolysiloxane, notamment en présence de catalyseur organopéroxyde ; ou par réticulation d'organopolysiloxane par radiations de haute énergie telles que rayons gamma, rayons ultraviolet, faisceau électronique.

De préférence, l'organopolysiloxane réticulé élastomère est obtenu par réaction d'addition réticulation (A) de diorganopolysiloxane contenant au moins deux hydrogènes liés chacun à un silicium, et (B) de diorganopolysiloxane ayant au moins deux groupements à insaturation éthylénique (notamment groupes vinyliques) liés au silicium, notamment en présence (C) de catalyseur platine, comme par exemple décrit dans la demande EP-A-295886.

En particulier, l'organopolysiloxane peut être obtenu par réaction de diméthylpolysiloxane à terminaisons diméthylvinylsiloxy et de méthylhydrogénopolysiloxane à terminaisons triméthylsiloxy, en présence de catalyseur platine.

Le composé (A) est le réactif de base pour la formation d'organopolysiloxane élastomère et la réticulation s'effectue par réaction d'addition du composé (A) avec le composé (B) en présence du catalyseur (C).

Le composé (A) est en particulier un organopolysiloxane ayant au moins 2 atomes d'hydrogène liés à des atomes de silicium distincts dans chaque molécule.

Le composé (A) peut présenter toute structure moléculaire, notamment une structure chaîne linéaire ou chaîne ramifiée ou une structure cyclique.

Le composé (A) peut avoir une viscosité à 25 °C allant de 1 à 50 000 centistokes, notamment pour être bien miscible avec le composé (B).

Les groupes organiques liés aux atomes de silicium du composé (A) peuvent être des groupes alkyles tels que méthyl, éthyl, propyl, butyl, octyl ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto.

Le composé (A) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane.

Le composé (B) est avantageusement un diorganopolysiloxane ayant au moins deux groupes alkényles inférieur (par exemple en C2-C4) ; le groupe alkényle inférieur peut être choisi parmi les groupes vinyl, allyl, et propényl. Ces groupements alkényles inférieurs peuvent être situés en toute position de la molécule organopolysiloxane mais sont de préférence situés aux extrémité de la molécule organopolysiloxane. L'organopolysiloxane (B) peut avoir une structure chaîne ramifiée, chaîne linéaire, cyclique ou réseau mais la structure chaîne linéaire est préférée. Le composé (B) peut avoir une viscosité allant de l'état liquide à l'état de gomme. De préférence, le composé (B) a une viscosité d'au moins 100 centistokes à 25 °C.

Les organopolysiloxanes (B) peuvent être choisi parmi les méthylvinylsiloxanes, les copolymères méthylvinylsiloxane-diméthylsiloxane, les diméthylpolysiloxanes à terminaisons diméthylvinylsiloxy, les copolymères diméthylsiloxane-méthylphénylsiloxane à terminaisons diméthylvinylsiloxy, les copolymères diméthylsiloxane-diphénylsiloxane-méthylvinylsiloxane à terminaisons diméthylvinylsiloxy, les copolymères diméthylsiloxane-méthylvinylsiloxane à terminaisons triméthylsiloxy, les copolymères diméthylsiloxane-méthylphénylsiloxane-méthylvinylsiloxane à terminaisons triméthylsiloxy, les méthyl(3,3,3-trifluoropropyl)polysiloxane à terminaisons diméthylvinylsiloxy, et les copolymères diméthylsiloxane-méthyl(3,3,3-trifluoropropyl) siloxane à terminaisons diméthylvinylsiloxy.

Outre les groupes alkényle précités, les autres groupes organiques liés aux atomes de silicium dans le composé (B) peuvent être des groupes alkyles tels que méthyl, éthyl, propyl, butyl ou octyl ; des groupes alkyles substitués tels que 2-phényléthyl, 2-phénylpropyl ou 3,3,3-trifluoropropyl ; des groupes aryles tels que phényl, tolyl ou xylyl ; des groupes aryles substitués tels que phényléthyl ; et des groupes hydrocarbonés monovalents substitués tels qu'un groupe époxy, un groupe ester carboxylate, ou un groupe mercapto.

Les organopolysiloxanes (B) peuvent être choisis parmi les méthylvinylpolysiloxanes, les copolymères méthylvinylsiloxane-diméthylsiloxane, les diméthylpolysiloxanes à terminaisons diméthylvinylsiloxy, les copolymères diméthylsiloxane-méthylphénylsiloxane à terminaisons diméthylvinylsiloxy, les copolymères diméthylsiloxane-diphénylsiloxane-méthylvinylsiloxane à terminaisons diméthylvinylsiloxy, les copolymères diméthylsiloxane-méthylvinylsiloxane à terminaisons triméthylsiloxy, les copolymères diméthylsiloxane-méthylphénylsiloxane-méthylvinylsiloxane à terminaisons triméthylsiloxy, les méthyl(3,3,3-trifluoropropyl)polysiloxane à terminaisons diméthylvinylsiloxy, et les copolymères diméthylsiloxane-méthyl(3,3,3-trifluoropropyl)siloxane à terminaisons diméthylvinylsiloxy.

En particulier, l'organopolysiloxane élastomère peut être obtenu par réaction de diméthylpolysiloxane à terminaisons diméthylvinylsiloxy et de méthylhydrogénopolysiloxane à terminaisons triméthylsiloxy, en présence de catalyseur platine.

Avantageusement, la somme du nombre de groupements éthyléniques par molécule du composé (B) et du nombre d'atomes d'hydrogène liés à des atomes de silicium par molécule du composé (A) est d'au moins 5.

Il est avantageux que le composé (A) soit ajouté en une quantité telle que le rapport moléculaire entre la quantité totale d'atomes d'hydrogène liés à des atomes de silicium dans le composé (A) et la quantité totale de tous les groupements à insaturation éthylénique dans le composé (B) soit compris dans la gamme de 1,5/1 à 20/1.

Le composé (C) est le catalyseur de la réaction de réticulation , et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C) est de préférence ajouté de 0,1 à 1000 parts en poids, mieux de 1 à 100 parts en poids, en tant que métal platine propre pour 1000 parts en poids de la quantité totale des composés (A) et (B).

De telles particules d'organopolysiloxane réticulé élastomère sont notamment décrites dans les demandes JP-A-61-194009, EP-A-242219, EP-A-381166.

Avantageusement, l'élastomère est un élastomère non-émulsionnant. Le terme "non émulsionnant" définit des élastomères organopolysiloxane ne contenant pas de motifs polyoxyalkylène.

Comme élastomères sous forme de poudre, on peut utiliser ceux vendus sous les dénominations "DC9505", "DC 9506" par la société Dow Corning,

Selon un mode particulier de réalisation de l'invention, les particules d'organopolysiloxane réticulé élastomère utilisées peuvent se présenter sous la forme d'une dispersion aqueuse.

Les organopolysiloxanes élastomériques selon l'invention peuvent être choisis parmi les polymères réticulés décrits dans la demande JP-A-10/175816 ou le brevet US 5928660. Selon cette demande, ils sont obtenus par réaction d'addition et de réticulation, en présence d'un catalyseur en particulier du type platine, d'au moins :

- (a) un organopolysiloxane (i) ayant au moins deux groupes vinyliques en position $\alpha$-$\omega$ de la chaîne siliconée par molécule ; et
- (b) un organosiloxane (ii) ayant au moins un atome d'hydrogène lié à un atome de silicium par molécule.

En particulier, l'organopolysiloxane (i) est choisi parmi les polydiméthylsiloxanes et est plus spécifiquement un $\alpha$-$\omega$-diméthylvinyl polydiméthylsiloxane.

Les organopolysiloxanes élastomériques de la composition selon l'invention se présente avantageusement sous forme de suspension aqueuse. Cette suspension peut être notamment obtenue comme suit :

- (a) mélange d'un organopolysiloxane (i) ayant au moins deux groupes vinyliques en position $\alpha$-$\omega$ de la chaîne siliconée par molécule et d'un organosiloxane (ii) ayant au moins un atome d'hydrogène lié à un atome de silicium par molécule ;
- (b) ajout d'un catalyseur, en particulier de type platine ;
- (b) ajout d'une phase aqueuse contenant un émulsifiant pour former une émulsion ;
- (c) polymérisation de l'organopolysiloxane (i) et de l'organosiloxane (ii) en émulsion en présence d'un catalyseur de platine.

L'émulsionnant peut être choisis parmi les tensioactifs non ioniques, cationiques ou anioniques de HLB $\geq$ 8, de préférence choisi parmi les tensioactifs non ioniques. La proportion de tensioactifs est de préférence de 0,1 à 20 parties en poids pour 100 parties en poids de la composition d'organopolysiloxane élastomérique, et mieux, de 0,5 à 10 parties en poids (cf. description du document JP-A-10/175816).

Après l'étape (c), il est possible de sécher les particules obtenues, pour en évaporer toute ou partie de l'eau piégée.

Les organopolysiloxanes peuvent être sous la forme de particules solides déformables ayant une certaine dureté, mesurable avec un duromètre Shore A (selon la norme ASTM D2240) à la température ambiante ou avec la méthode japonaise JIS-A. Cette dureté peut être mesurée sur un bloc d'élastomère préparé à cet effet comme suit : mélange de l'organopolysiloxane (i) et de l'organosiloxane (ii) ; élimination de l'air du mélange ; moulage et vulcanisation au four à 100°C pendant 30 minutes ; refroidissement à température ambiante puis mesure de la dureté. La densité est aussi déterminée sur ce bloc d'élastomère.

L'organopolysiloxane peut avoir une dureté JIS-A inférieure ou égale à 80, notamment allant de 10 à 80), et de préférence inférieure ou égale à 65, notamment allant de 15 à 65.

Comme particules d'organopolysiloxane en dispersion dans l'eau, on peut utiliser celles commercialisées sous les noms BY 29-122 et BY 29-119 par la société Dow-Corning.

Les particules d'organopolysiloxane réticulé élastomère peuvent être présentes dans la composition selon l'invention en une teneur allant de 20 % à 50 % en poids, par rapport au poids total de la composition, de préférence allant de 30 % à 50 % en poids, préférentiellement allant de 35 % à 45 % en poids, et plus préférentiellement allant de 35 % à 40 % en poids.

La composition selon l'invention contient au moins des particules additionnelles différentes des particules d'organopolysiloxane réticulé élastomère décrites précédemment.

Les particules additionnelles peuvent être choisies parmi les matières colorantes pulvérulentes, les charges, les fibres, et leurs mélanges. La composition selon l'invention peut donc comprendre des matières colorantes et/ou des charges et/ou des fibres.

La matière colorante pulvérulente peut être notamment choisie parmi les pigments, les nacres, et leurs mélanges.

Par pigments, il faut comprendre des particules de toute forme, blanches ou colorées, minérales ou organiques, insolubles dans le milieu physiologique, destinées à colorer la composition.
Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.

Les matières colorantes pulvérulentes peuvent être présentes dans la composition selon l'invention en une teneur allant de 0,5 % à 50 % en poids, par rapport au poids total de la composition, de préférence allant de 1 % à 30 % en poids.

La composition selon l'invention peut comprendre des charges.

Par charges, il faut comprendre des particules de toute forme, incolores ou blanches, minérales ou de synthèse, insolubles dans le milieu de la composition quelle que soit la température à laquelle la composition est fabriquée.

Les charges peuvent être minérales ou organiques de toute forme, plaquettaires, sphériques ou oblongues, quelle que soit la forme cristallographique ( par exemple feuillet, cubique, hexagonale, orthorombique, etc). On peut citer le talc, le mica, la silice, le kaolin, les poudres de polyamide (Nylon®) , de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique, les poudres de copolymère diméthacrylate d'éthylèneglycol et de méthacrylate de lauryle notamment vendues sous la dénomination POLYTRAP® 6603 adsorber par la société RP Scherer, les microbilles de résine de silicone (Tospearls® de Toshiba, par exemple), le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

De préférence, les charges sont choisies parmi le mica, les poudres de polyamide, les poudres de copolymère diméthacrylate d'éthylèneglycol et de méthacrylate de lauryle, le nitrure de bore, et leurs mélanges.

Les charges peuvent être présentes dans la composition en une teneur allant de 0,5 % à 40 % en poids, par rapport au poids total de la composition, de préférence allant de 1 % à 35 % en poids, préférentiellement allant de 5 % à 30 % en poids, et plus préférentiellement allant de 15 % à 30 % en poids.

La composition selon l'invention peut comprendre des fibres.

Par "fibre", il faut comprendre un objet de longueur L et de diamètre D tel que L soit très supérieur à D, D étant le diamètre du cercle dans lequel s'inscrit la section de la fibre. En particulier, le rapport UD (ou facteur de forme) est choisi dans la gamme allant de 3,5 à 2500, de préférence de 5 à 500, et mieux de 5 à 150.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 μm à 10 mm, de préférence de 0,1 mm à 5 mm et mieux de 0,3 mm à 1 mm . Leur section peut être comprise dans un cercle de diamètre allant de 2 nm à 500 μm, de préférence allant de 100 nm à 100 μm et mieux de 1 μm à 50 μm. Le poids ou titre des fibres est souvent donné en denier ou décitex et représente le poids en gramme pour 9 km de fil. De préférence, les fibres selon l'invention ont un titre choisi dans la gamme allant de

0,01 à 10 à deniers, de préférence de 0,1 à 2 deniers et mieux de 0,3 à 0,7 deniers.

Les fibres peuvent être celles utilisées dans la fabrication des textiles et notamment des fibres de soie, de coton, de laine, de lin, des fibres de cellulose - notamment extraites notamment du bois, des légumes ou des algues -, de rayonne, de polyamide (Nylon®), de viscose, d'acétate notamment d'acétate de rayonne, de poly-(p-phénylène-téréphtalamide) (ou d'aramide) notamment de Kevlar®, de polymère acrylique notamment de polyméthacrylate de méthyle ou de poly 2-hydroxyéthyl méthacrylate, de polyoléfine et notamment de polyéthylène ou de polypropylène, de verre, de silice, de carbone notamment sous forme graphite, de polytétrafluoroéthylène (comme le Téflon®), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester.

De préférence, les fibres sont des fibres de polyamide (Nylon®).

On peut aussi utiliser les fibres utilisées en chirurgie comme les fibres synthétiques résorbables préparées à partir d'acide glycolique et caprolactone ("Monocryl" de chez Johnson & Johnson) ; les fibres synthétiques résorbables du type copolymère d'acide lactique et d'acide glycolique ("Vicryl" de chez Johnson & Johnson) ; les fibres de polyester téréphtalique ("Ethibond" de chez Johnson & Johnson) et les fils d'acier inoxydable ("Acier" de chez Johnson & Johnson).

Par ailleurs, les fibres peuvent être traités ou non en surface, enrobées ou non d'une couche de protection.

Comme fibres enrobées utilisables dans l'invention, on peut citer des fibres de polyamides enrobées de sulfure de cuivre pour un effet anti-statique (par exemple le "R-STAT" de chez Rhodia) ou un autre polymère permettant une organisation particulière des fibres (traitement de surface spécifique). On peut encore citer les fibres enrobées par des pigments minéraux ou organiques, tels que les pigments cités plus loin dans la demande.

De préférence, on utilise des fibres d'origine synthétique et en particulier des fibres organiques, comme celles utilisées en chirurgie.

Les fibres utilisables dans la composition selon l'invention sont préférentiellement des fibres de polyamide, de cellulose, de poly-p-phénylène téréphtalamide ou de de polyéthylène. Leur longueur (L) peut aller de 0,1 mm à 5 mm, de préférence de 0,25 mm à 1,6 mm et leur diamètre moyen peut aller de 1 µm à 50 µm. En particulier, on peut utiliser les fibres de polyamide commercialisées par les Etablissements P. Bonte sous le nom de "Polyamide 0.9 Dtex 3 mm", ayant un diamètre moyen de 6 µm, un titre d'environ 0.9 dtex et une longueur allant de 0,3 mm à 5 mm, ou bien encore les fibres de polyamides vendues sous la dénomination Fiber-Ion 931-D1-S par la société LCW , ayant un titre d'environ 0,9 dtex et une longueur d'environ 0,3 mm. On peut aussi utiliser les fibres de celluloses (ou de rayonne) ayant un diamètre moyen de 50 µm et une longueur allant de 0,5 mm à 6 mm comme celles vendues sous le nom de "Natural rayon flock fiber RC1BE - N003 - M04" par la société Claremont Flock. On peut également utiliser des fibres de polyéthylène comme celles vendues sous le nom de "Shurt Stuff 13 099 F" par la société Mini Fibers.

La composition selon l'invention peut également comprendre des fibres dites "rigides", par opposition aux fibres citées précédemment, qui ne sont pas des fibres rigides.

Les fibres rigides, initialement sensiblement droites, lorsqu'elles sont placées dans un milieu dispersant, ne voient pas leur forme sensiblement modifiée, ce qui se traduit par la condition angulaire définie ci-après, reflétant une forme que l'on peut qualifier de toujours, sensiblement droite, linéaire. Cette condition d'angle reflète la rigidité des fibres qui peut difficilement être exprimée par un autre paramètre pour des objets ayant une taille aussi faible que les fibres rigides.

La rigidité des fibres se traduit par la condition angulaire suivante : avantageusement, au moins 50 % en nombre, de préférence au moins 75 % en nombre, et mieux au moins 90 % en nombre des fibres sont telles que l'angle formé entre la tangente à l'axe central longitudinal de la fibre et la droite reliant ladite extrémité au point sur l'axe central longitudinal de la fibre correspondant à la moitié de la longueur de la fibre soit inférieur à 15 ° et l'angle formé entre la tangente à l'axe central longitudinal de la fibre en un point situé à mi-longueur de la fibre et la droite reliant l'une des extrémités au point sur l'axe central longitudinal de la fibre correspondant à la moitié de la longueur de fibre soit inférieur ou égal à 15 °, pour une même longueur de fibre allant de 0,8 mm à 5 mm, de préférence allant de 1 mm à 4 mm, de préférence allant de 1 mm à 3 mm, et mieux de 2 mm.

Avantageusement, l'angle, mentionné ci-dessus, est mesuré aux deux extrémités de la fibre et en un point situé à mi-longueur de la fibre, en d'autres termes, on effectue dans ce cas trois mesures et la moyenne des angles mesurés est inférieure ou égale à 15°.

Notamment, la tangente, en tout point de la fibre, forme un angle inférieur à 15°.

Dans la présente demande, l'angle formé par la tangente en un point de la fibre est l'angle formé entre la tangente à l'axe central longitudinal de la fibre audit point de la fibre et la droite reliant l'extrémité de la fibre la plus proche dudit point au point sur l'axe central longitudinal de la fibre correspondant à la moitié de la longueur de la fibre.

Généralement, les fibres rigides utilisables dans la composition selon l'invention ont la même longueur de fibre ou une longueur sensiblement identique.

Plus précisément lorsque l'on observe au microscope, avec un objectif permettant un grossissement

de 2,5 et avec une vision plein champ, un milieu dans lequel sont dispersées les fibres rigides à une concentration des fibres de 1 % en poids, un nombre majoritaire de fibres rigides, c'est-à-dire au moins 50 % en nombre des fibres rigides, de préférence au moins 75 % en nombre des fibres rigides, et mieux au moins 90 % en nombre des fibres rigides, doivent satisfaire à la condition angulaire définie plus haut. La mesure conduisant à la valeur de l'angle est effectuée pour une même longueur de fibres, cette longueur est comprise dans la gamme allant de 0,8 mm à 5 mm, de préférence de 1 à 4 mm, de préférence de 1 à 3 mm, et mieux de 2 mm.

Le milieu dans lequel est réalisée l'observation est un milieu dispersant assurant une bonne dispersion des fibres rigides, par exemple de l'eau, un gel aqueux d'argile ou de polyuréthane associatif. On peut même réaliser une observation directe de la composition contenant les fibres rigides. Un échantillon de la composition ou de la dispersion préparée est placée entre lame et lamelle pour l'observation au microscope avec un objectif permettant un grossissement de 2,5 et avec une vision plein champ. La vision plein champ permet de voir les fibres dans leur intégralité.

Les fibres rigides peuvent être choisies parmi les fibres d'un polymère synthétique choisi parmi les polyesters, les polyuréthanes, les polymères acryliques, les polyoléfines, les polyamides, en particulier les polyamides non aromatiques et les polyimides-amides aromatiques.

Comme exemples de fibres rigides, on peut citer les fibres :

- de polyesters, telles que celles obtenues par découpe de fils vendus sous les dénominations FIBRE 255-100-R11-242T TAILLE 3 MM (section octalobée), FIBRE 265-34-R11-56T TAILLE 3 MM (section ronde), FIBRE COOLMAX 50-34-591 TAILLE 3 MM (section tétralobée) par la société DUPONT DE NEMOURS ;

- de polyamide, telles que celles vendues sous les dénominations TRILOBAL NYLON 0.120-1.8 DPF ; TRILOBAL NYLON 0.120-18 DPF ; NYLON 0.120-6 DPF par la société Cellusuede products ; ou obtenues par découpe de fils vendus sous la dénomination FIBRE NOMEX BRAND 430 TAILLE 3 MM par la société DUPONT DE NEMOURS ;

- de polyimide-amide telles que celles vendues sous les dénomination "KERMEL", " KERMEL TECH" par la société RHODIA ;

- de poly-(p-phénylène-téréphtalamide) (ou d'aramide) notamment vendues sous la dénomination Kevlar ® par la société DUPONT DE NEMOURS ;

- des fibres à structure multicouche comprenant des couches alternées de polymères choisis parmi les polyesters, les polymères acryliques, les polyamides, tellles que celles décrites dans les documents EP-A-6921217, EP-A-686858 et US-A-5472798. De telles fibres sont vendues sous les dénominations "Morphotex", « Teijin Tetron Morphotex » par la société TEIJIN.

Les fibres rigides particulièrement préférées sont les fibres de polyimide-amide aromatiques.

Des fils ou fibres de polyimide-amide, qui peuvent être utilisés pour les compositions de l'invention, sont décrits, par exemple, dans le document de R. PIGEON et P. ALLARD, Chimie Macromoléculaire Appliquée, 40/41 (1974), pages 139-158 (n° 600), ou bien encore dans les documents US-A-3 802 841, FR-A-2 079 785, EP-A1-0 360 728, EP-A-0 549 494, auxquels on pourra se référer.

Les fibres de polyimide-amide aromatique préférées sont des fibres de polyimide-amide comprenant des motifs répétitif de formule :

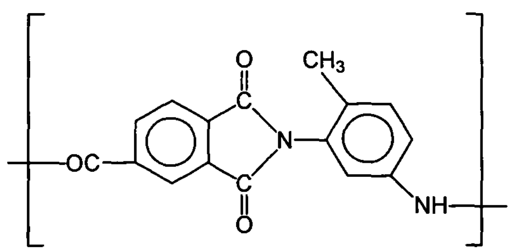

obtenu par polycondensation du toluylène diisocyanate et de l'anhydride triméllitique.

Les fibres peuvent êtres présentes dans la composition selon l'invention en une teneur allant de 0,5 % à 20 % en poids, par rapport au poids total de la composition, de préférence allant de 0,5 % à 15 % en poids, préférentiellement allant de 0,5 % à 8 % en poids, et plus préférentiellement allant de 2 % à 8 % en poids.

Avantageusement, la composition selon l'invention peut comprendre une teneur totale en particules additionnelles (donc matière colorante + charges + fibres) allant de 20 % à 50 % en poids, par rapport au poids total de la composition, de préférence allant de 25 % à 45 % en poids, préférentiellement allant de 25 % à 40 % en poids, et plus préférentiellement allant de 30 % à 40 % en poids.

De préférence, les particules d'organopolysiloxane réticulé élastomère et les particules additionnelles sont présentes dans la composition selon l'invention en des teneurs telles que le rapport pondéral particules d'organopolysiloxane réticulé/particules additionnelles va de 1,21 à 2, de préférence va de 1,21 à 1,5, préférentiellement va de 1,21 à 1,3.

La composition selon l'invention comprend de l'eau. L'eau peut être une eau florale telle que l'eau de bleuet et/ou une eau minérale telle que l'eau de VITTEL,

l'eau de LUCAS ou l'eau de LA ROCHE POSAY et/ou une eau thermale.

La composition peut comprendre de l'eau en une teneur allant de 5 % à 30 % en poids, par rapport au poids total de la composition, de préférence allant de 10 % à 25 % en poids, préférentiellement allant de 15 % à 25 % en poids, et plus préférentiellement allant de 20 % à 25 % en poids.

La composition peut comprendre également un polyol ayant notamment de 2 à 20 atomes de carbones, de préférence ayant de 2 à 10 atomes de carbone, et préférentiellement ayant de 2 à 6 atomes de carbones. Comme polyol, on peut citer la glycérine, le propylène glycol, le butylène glycol, le pentylène glycol, l'hexylène glycol, le dipropylène glycol, le diéthylène glycol, et leurs mélanges.

La composition selon l'invention peut comprendre un polyol en une teneur allant de 1 % à 20 % en poids, par rapport au poids total de la composition, et de préférence allant de 3 % à 15 % en poids.

La composition peut contenir d'autres ingrédients cosmétiques usuels pouvant être choisis notamment parmi les antioxydants, les parfums, les conservateurs, les neutralisants, les tensioactifs, les cires, les huiles, les filtres solaires, les vitamines, les hydratants, les composés auto-bronzants, les actifs antirides.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Avantageusement, la composition selon l'invention peut avoir une dureté allant de 0,07 N à 0,4 N, de préférence allant de 0,1 à 0,35 N et une élasticité EL allant de 15 % à 80 %, et de préférence allant de 30 % à 70 %.

La dureté et l'élasticité du produit sont mesurés à 20 °C à l'aide du texturomètre vendu sous la dénomination TA-TX2i par la société RHEO, équipé d'un mobile en inox en forme de bille de diamètre 12,7 mm en mesurant l'évolution de la force (force de compression ou force d'étirement) (F) en fonction du temps, pendant l'opération suivante :

> Le mobile est déplacé à la vitesse de 0,1 mm /s puis pénètre dans le produit jusqu'à une profondeur de pénétration de 0,3 mm. Lorsque le mobile a pénétré dans le produit à la profondeur de 0,3 mm, le mobile est retiré à la vitesse de 0,1 mm/s. Pendant le retrait du mobile, la force (force de compression) décroît fortement jusqu'à devenir nulle au bout d'un temps t . Pendant l'opération, le mobile effectue un aller retour en 6 secondes.

La dureté correspond à la force maximale de compression mesurée pendant l'opération ; elle est exprimée en Newton.

L'élasticité EL exprimée en pourcentage est déterminée par la relation :

$$EL\ (\%) = 100 \times (t-3)/(6-3)$$

La composition selon l'invention peut être préparée en mélangeant les divers ingrédients, notamment soit dans un mélangeur/granulateur à turbine de type Baker-Perkins, soit dans un malaxeur bi-vis continue type extrudeur-malaxeur BC21 de la société CLEXTRAL.

La composition peut être conditionnée dans une coupelle ou un boîtier par pressage du mélange des ingrédients.

Avantageusement, la composition selon l'invention ne contient pas le mélange suivant (les pourcentages étant exprimés en poids du poids total de la composition) :

8,1 % de dioxyde de titane
1,9 % d'oxydes de fer
10 % de poudre de nylon
5 % de fibres de polyamide
5 % de poudre acrylique
37,1 % de particules organopolysiloxane élastomère dispersées dans 21,8 % d'eau
5 % de glycérine
5 % de propylène glycol
1 % de conservateur,

L'invention est illustrée plus en détails par les exemples décrits ci-après.

**<u>Exemple 1</u> :**

On a préparé un produit de maquillage du teint ayant la composition suivante :

- Particules de polydiméthylsiloxane réticulé en dispersion aqueuse à 63 % en poids de polymère réticulé (BY 29-119 de Dow Corning) 59 g soit 37,1g MA
- Glycérine 5 g
- Propylène glycol 5 g
- Conservateurs 1 g
- Poudre de nylon (Orgasol® 2002 extra D NAT COS d'ATOFINA) 10 g
- Poudre de copolymère diméthacrylate d'éthylèneglycol et de méthacrylate de lauryle vendue sous la dénomination POLYTRAP® 6603 adsorber par la société RP Scherer 5 g
- Fibres de polyamide 0,9 Dtex de longueur 0,3 mm de la société Paul Bonte 5 g
- Pigments (oxydes de fer, dioxyde de titane) 10 g

Après mélange des ingrédients, le produit est conditionné dans une coupelle par pressage. Le produit

s'applique facilement sur la peau et permet d'obtenir un maquillage réparti de manière homogène.

Le produit obtenu a une dureté de 0,2 N et une élasticité de 55 % mesurées selon les conditions décrites précédemment.

**Exemple 2 :**

On a préparé un produit matifiant pour la peau ayant la composition suivante :

- Dispersion aqueuse de particules de polydiméthylsiloxane
  réticulé à 63 % en poids de polymère réticulé
  (BY 29-119 de Dow Corning) 59 g soit 37,1 g MA
- Glycérine 5 g
- Propylène glycol 5 g
- Conservateurs 1 g
- Poudre de nylon (Orgasol® 2002 extra D NAT COS d'ATOFINA) 20 g
- Poudre de copolymère diméthacrylate d'éthylèneglycol et de méthacrylate de lauryle vendue sous la dénomination POLYTRAP® 6603 adsorber par la société RP Scherer 5 g
- Fibres de polyamide 0,9 Dtex de longueur 0,3 mm de la société Paul Bonte 5 g

Après mélange des ingrédients, le produit est conditionné dans une coupelle par pressage. Le produit s'applique facilement sur la peau.

**Exemple 3 :**

On a préparé un produit de maquillage du teint ayant la composition suivante :

- Dispersion aqueuse de particules de polydiméthylsiloxane
  réticulé à 63 % en poids de polymère réticulé
  (BY 29-119 de Dow Corning) 59 g soit 37,1 g MA
- Glycérine 5 g
- Propylène glycol 5 g
- Conservateurs 1 g
- Poudre de nylon (Orgasol® 2002 extra D NAT COS d'ATOFINA) 15 g
- Poudre de copolymère diméthacrylate d'éthylèneglycol et de méthacrylate de lauryle vendue sous la dénomination POLYTRAP® 6603 adsorber par la société RP Scherer 5 g
- Pigments (oxydes de fer, dioxyde de titane) 10 g

Après mélange des ingrédients, le produit est conditionné dans une coupelle par pressage. Le produit s'applique facilement sur la peau et permet d'obtenir un maquillage homogène.

**Exemple 4 :**

On a préparé un produit matifiant pour la peau ayant la composition suivante :

- Dispersion aqueuse de particules de polydiméthylsiloxane
  réticulé à 63 % en poids de polymère réticulé
  (BY 29-119 de Dow Corning) 59 g soit 37,1g MA
- Glycérine 5 g
- Propylène glycol 5 g
- Conservateurs 1 g
- Poudre de nylon (Orgasol® 2002 extra D NAT COS d'ATOFINA) 25 g
- Poudre de copolymère diméthacrylate d'éthylèneglycol et de méthacrylate de lauryle vendue sous la dénomination
  POLYTRAP® 6603 adsorber par la société RP Scherer 5 g

Après mélange des ingrédients, le produit est conditionné dans une coupelle par pressage. Le produit s'applique facilement sur la peau.

**Exemple 5 :**

On a préparé un produit de maquillage du teint ayant la composition suivante :

- Dispersion aqueuse de particules de polydiméthylsiloxane
  réticulé à 63 % en poids de polymère réticulé
  (BY 29-119 de Dow Corning) 59 g soit 37,1 g MA
- Glycérine 5 g
- Propylène glycol 5 g
- Conservateurs 1 g
- Poudre de nylon (Orgasol® 2002 extra D NAT COS d'ATOFINA) 10 g
- Pigments (oxydes de fer, dioxyde de titane) 20 g

Après mélange des ingrédients, le produit est conditionné dans une coupelle par pressage. Le produit s'applique facilement sur la peau et permet d'obtenir un maquillage homogène.

**Exemple 6 :**

On a préparé un produit matifiant pour la peau ayant la composition suivante :

- Dispersion aqueuse de particules de polydiméthylsiloxane
  réticulé à 63 % en poids de polymère réticulé
  (BY 29-119 de Dow Corning) 59 g soit 37,1g MA

- Glycérine 5 g
- Propylène glycol 5 g
- Conservateurs 1 g
- Poudre de nylon (Orgasol® 2002 extra D NAT COS d'ATOFINA) 30 g

Après mélange des ingrédients, le produit est conditionné dans une coupelle par pressage. Le produit s'applique facilement sur la peau..

**Exemple 7 :**

On a préparé un fard à paupières ayant la composition suivante :

- Particules de polydiméthylsiloxane réticulé réticulé (DC9506 de Dow Corning) 42g
- Eau 15g
- Glycérine 10 g
- Conservateurs 1 g
- Poudre de copolymère diméthacrylate d'éthylèneglycol et de méthacrylate de lauryle vendue sous la dénomination POLYTRAP® 6603 adsorber par la société RP Scherer 2 g
- Nacres 30 g

Après mélange des ingrédients, le produit est conditionné dans une coupelle par pressage. Le produit s'applique facilement sur la peau.

**Exemple 8 :**

On a préparé un produit de maquillage du teint ayant la composition suivante :

- Particules de polydiméthylsiloxane réticulé réticulé (DC9506 de Dow Corning) 40g
- Eau 19g
- Glycérine 5 g
- Propylène glycol 5 g
- Conservateurs 1 g
- Poudre de nylon (Orgasol® 2002 extra D NAT COS d'ATOFINA) 10 g
- Poudre de copolymère diméthacrylate d'éthylèneglycol et de méthacrylate de lauryle vendue sous la dénomination POLYTRAP® 6603 adsorber par la société RP Scherer 5 g
- Fibres de polyamide 0,9 Dtex de longueur 0,3 mm de la société Paul Bonte 5 g
- Pigments (oxydes de fer, dioxyde de titane) 10 g

Après mélange des ingrédients, le produit est conditionné dans une coupelle par pressage. Le produit s'applique facilement sur la peau et permet d'obtenir un maquillage homogène.

**Exemple 9 :**

On a préparé un produit matifiant pour la peau ayant la composition suivante :

- Particules de polydiméthylsiloxane réticulé réticulé (DC9506 de Dow Corning) 40g
- Eau 19g
- Glycérine 5 g
- Propylène glycol 5 g
- Conservateurs 1 g
- Poudre de nylon (Orgasol® 2002 extra D NAT COS d'ATOFINA) 20 g
- Poudre de copolymère diméthacrylate d'éthylèneglycol et de méthacrylate de lauryle vendue sous la dénomination - POLYTRAP® 6603 adsorber par la société RP Scherer 5 g
- Fibres de polyamide 0,9 Dtex de longueur 0,3 mm de la société Paul Bonte 5 g

Après mélange des ingrédients, le produit est conditionné dans une coupelle par pressage. Le produit s'applique facilement sur la peau.

## Revendications

1. Composition poudreuse comprenant de l'eau, des particules solides d'organopolysiloxane réticulé élastomère, une poudre additionnelle, les particules d'organopolysiloxane réticulé élastomère et la poudre additionnelle étant présentes selon un rapport pondéral organopolysiloxane réticulé élastomère/poudre additionnelle supérieur à 1,2 et inférieur ou égal à 2,5.

2. Composition selon la revendication précédente, **caractérisée par le fait que** l'organopolysiloxane réticulé est choisi parmi ceux obtenus :

   - par réaction d'addition réticulation de diorganosiloxane contenant au moins un hydrogène lié au silicium et de diorganopolysiloxane ayant des groupements à insaturation éthylénique liés au silicium ;
   - par réaction de condensation réticulation déhydrogénation entre un diorganopolysiloxane à terminaisons hydroxyle et un diorganopolysiloxane contenant au moins un hydrogène lié au silicium ;
   - par réaction de condensation réticulation d'un diorganopolysiloxane à terminaisons hydroxyle et d'un organopolysilane hydrolysable ;
   - par réticulation thermique d'organopolysiloxane ;
   - par réticulation d'organopolysiloxane par radiations de haute énergie.

**3.** Composition selon la revendication 1 ou 2, **caractérisée par le fait que** l'organopolysiloxane réticulé est obtenu par réaction d'addition réticulation (A) de diorganopolysiloxane contenant au moins deux hydrogènes liés chacun à un silicium, et (B) de diorganopolysiloxane ayant au moins deux groupements à insaturation éthylénique liés au silicium, notamment en présence (C) de catalyseur platine.

**4.** Composition selon l'une quelconque des revendications 1 à 3, **caractérisée par le fait que** l'organopolysiloxane réticulé est obtenu par réaction de diméthylpolysiloxane à terminaisons diméthylvinylsiloxy et de méthylhydrogénopolysiloxane à terminaisons triméthylsiloxy, en présence de catalyseur platine.

**5.** Composition selon l'une quelconque des revendications précédentes, **caractérisée par le fait que** les particules d'organopolysiloxane réticulé élastomère sont présentes en une teneur allant de 20 % à 50 % en poids, par rapport au poids total de la composition, de préférence allant de 30 % à 50 % en poids, préférentiellement allant de 35 % à 45 % en poids, et plus préférentiellement allant de 35 % à 40 % en poids.

**6.** Composition selon l'une quelconque des revendications précédentes, **caractérisée par le fait que** les particules additionnelles sont choisies parmi les matières colorantes pulvérulentes, les charges, les fibres, et leurs mélanges.

**7.** Composition selon l'une quelconque des revendications précédentes, **caractérisée par le fait que** les particules additionnelles comprennent une matière colorante pulvérulente.

**8.** Composition selon la revendications 6 ou 7, **caractérisée par le fait que** la matière colorante pulvérulente est choisie parmi les pigments, les nacres et leurs mélanges.

**9.** Composition selon l'une quelconque des revendications 6 à 8, **caractérisée par le fait que** la matière colorante pulvérulente est choisie parmi le dioxyde de titane, les oxydes de zirconium, les oxydes de cérium, les oxydes de zinc, les oxydes de fer, les oxydes de chrome, le violet de manganèse, le bleu outremer, l'hydrate de chrome, le bleu ferrique, la poudre d'aluminium, la poudre de cuivre.

**10.** Composition selon l'une quelconque des revendications 6 à 9, **caractérisée par le fait que** la matière colorante pulvérulente est présente en une teneur allant de 0,5 % à 50 % en poids, par rapport au poids total de la composition, de préférence allant de 1 % à 30 % en poids.

**11.** Composition selon l'une quelconque des revendications précédentes, **caractérisée par le fait qu'**elle les particules additionnelles comprennent une charge.

**12.** Composition selon la revendication précédente, **caractérisée par le fait que** la charge est choisie parmi le talc, le mica, la silice, le kaolin, les poudres de polyamide, les poudres de poly-β-alanine, les poudres de polyéthylène, les poudres de polymères de tétrafluoroéthylène, la lauroyl-lysine, l'amidon, le nitrure de bore, les microsphères creuses de chlorure de polyvinylidène/acrylonitrile, les microsphères creuses de copolymères d'acide acrylique, les poudres de copolymère diméthacrylate d'éthylèneglycol et de méthacrylate de lauryle, les microbilles de résine de silicone, le carbonate de calcium précipité, le carbonate de magnésium, l'hydrocarbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, et leurs mélanges.

**13.** Composition selon la revendication 11 ou 12, **caractérisée par le fait que** la charge est choisie parmi le mica, les poudres de polyamide, les poudres de copolymère diméthacrylate d'éthylèneglycol et de méthacrylate de lauryle, le nitrure de bore, et leurs mélanges.

**14.** Composition selon l'une quelconque des revendications 11 à 13, **caractérisée par le fait que** la charge est présente en une teneur allant de 0,5 % à 40 % en poids, par rapport au poids total de la composition, de préférence allant de 1 % à 35 % en poids, préférentiellement allant de 5 % à 30 % en poids, et plus préférentiellement allant de 15 % à 30 % en poids.

**15.** Composition selon l'une quelconque des revendications précédentes, **caractérisée par le fait que** les particules additionnelles comprennent des fibres.

**16.** Composition selon la revendication précédente, **caractérisée en ce que** les fibres sont choisies parmi les fibres de soie, de coton, de laine, de lin, des fibres de cellulose, de polyamide, de viscose, d'acétate notamment d'acétate de rayonne, de poly-p-(phénylène-téréphtalamide), en acrylique notamment de polyméthacrylate de méthyle ou de poly 2-hydroxyéthyl méthacrylate, de polyoléfine et notamment de polyéthylène ou de polypropylène, de silice, de carbone notamment sous forme graphite, de polytétrafluoroéthylène, de collagène insoluble, de polyesters, de polychlorure de vinyle ou de vinylidène, d'alcool polyvinylique, de polyacrylonitrile,

de chitosane, de polyuréthane, de polyéthylène phtalate, des fibres de mélanges de polymères, les fibres rigides sensiblement rectilignes et leurs mélanges.

**17.** Composition selon l'une des revendications 15 ou 16, **caractérisée en ce que** les fibres sont des fibres d'origine synthétique.

**18.** Composition selon l'une quelconque des revendications 15 à 17, **caractérisée en ce que** les fibres ont une longueur allant de 1 µm à 10 mm, de préférence de 0,1 mm à 5 mm et mieux de 0,3 à 1 mm.

**19.** Composition selon l'une des revendications 15 à 18, **caractérisée en ce que** les fibres ont un section comprise dans un cercle de diamètre allant de 2 nm à 500 µm, de préférence allant de 100 nm à 100 µm.

**20.** Composition selon l'une quelconque des revendications 15 à 19, **caractérisée par le fait que** les fibres ont une longueur L et un diamètre D tel que L/D est choisi dans la gamme allant de 1,5 à 2 500, de préférence de 3,5 à 500 et mieux de 5 à 150.

**21.** Composition selon l'une quelconque des revendications 15 à 20, **caractérisée par le fait que** les fibres sont présentes en une teneur allant de 0,5 % à 20 % en poids, par rapport au poids total de la composition, de préférence allant de 0,5 % à 15 % en poids, préférentiellement allant de 0,5 % à 8 % en poids, et plus préférentiellement allant de 2 % à 8 % en poids.

**22.** Composition selon l'une quelconque des revendications précédentes, **caractérisée par le fait qu'**elle comprend une teneur totale en particules additionnelles allant de 20 % à 50 % en poids, par rapport au poids total de la composition, de préférence allant de 25 % à 45 % en poids, préférentiellement allant de 25 % à 40 % en poids, et plus préférentiellement allant de 30 % à 40 % en poids.

**23.** Composition selon l'une quelconque des revendications précédentes, **caractérisée par le fait que** les particules d'organopolysiloxane réticulé élastomère et les particules additionnelles sont présentes en des teneurs telles que le rapport pondéral particules d'organopolysiloxane réticulé/particules additionnelles va de 1,21 à 2, de préférence va de 1,21 à 1,5, préférentiellement va de 1,21 à 1,3.

**24.** Composition selon l'une quelconque des revendications précédentes, **caractérisée par le fait que** l'eau est présente en une teneur allant de 5 % à 30 % en poids, par rapport au poids total de la composition, de préférence allant de 10 % à 25 % en poids, préférentiellement allant de 15 % à 25 % en poids, et plus préférentiellement allant de 20 % à 25 % en poids.

**25.** Composition selon l'une quelconque des revendications précédentes, **caractérisée par le fait qu'**elle comprendre un polyol ayant notamment de 2 à 20 atomes de carbones, de préférence ayant de 2 à 10 atomes de carbone, et préférentiellement ayant de 2 à 6 atomes de carbones.

**26.** Composition selon la revendication précédente, **caractérisée par le fait que** le polyol est choisi parmi la glycérine, le propylène glycol, le butylène glycol, le pentylène glycol, l'hexylène glycol, le dipropylène glycol, le diéthylène glycol, et leurs mélanges.

**27.** Composition selon la revendication 25 ou 26, **caractérisée par le fait que** le polyol est présent en une teneur allant de 1 % à 20 % en poids, par rapport au poids total de la composition, et de préférence allant de 3 % à 15 % en poids.

**28.** Composition selon l'une quelconque des revendications précédentes, **caractérisée par le fait qu'**elle comprend un ingrédient cosmétique choisi parmi les antioxydants, les parfums, les conservateurs, les neutralisants, les tensioactifs, les cires, les huiles, les filtres solaires, les vitamines, les hydratants, les composés auto-bronzants, les actifs antirides.

**29.** Composition selon l'une quelconque des revendications précédentes, **caractérisée par le fait que** la composition a une dureté allant de 0,07 N à 0,4 N, de préférence allant de 0,1 à 0,35 N et a une élasticité EL allant de 15 % à 80 %, et de préférence allant de 30 % à 70 %.

**30.** Composition selon l'une quelconque des revendications précédentes, **caractérisée par le fait que** la composition est une composition cosmétique.

**31.** Composition selon l'une quelconque des revendications précédentes, **caractérisée par le fait que** la composition est sous la forme d'une poudre libre, d'une poudre compacte, d'un poudre pressée, d'une composition pâteuse.

**32.** Composition selon l'une quelconque des revendications précédentes, **caractérisée par le fait que** la composition est une composition de maquillage ou de soin des matières kératiniques, en particulier de la peau.

**33.** Composition selon l'une quelconque des revendications précédentes, **caractérisée par le fait que** la composition est un produit du teint (notamment fond de teint), un fard à paupières, un fard à joue, un produit anticernes, un produit de maquillage du

corps, un produit de maquillage des lèvres, un produit de maquillage des ongles, un produit de maquillage des ongles, un produit de maquillage des cheveux.

**34.** Composition selon l'une quelconque des revendications précédentes, **caractérisée par le fait que** la composition est une composition de maquillage de la peau, en particulier une composition de maquillage du teint, notamment un fond de teint.

**35.** Composition selon l'une quelconque des revendications 1 à 31, **caractérisée par le fait que** la composition de soin de la peau est un produit de soin de la peau, un produit matifiant de la peau, un produit de protection solaire de la peau, une composition autobronzante, un produit déodorant.

**36.** Procédé de maquillage des matière kératiniques, notamment de la peau, comprenant l'application sur les matières kératiniques, notamment sur la peau, d'une composition selon l'une quelconque des revendications précédentes.

**37.** Utilisation d'une composition selon l'une quelconque des revendications 1 à 35 pour obtenir un maquillage facile à appliquer sur les matières kératiniques, en particulier sur la peau, ou un maquillage homogène déposé sur les matières kératiniques, en particulier sur la peau.